# EUROPEAN PATENT SPECIFICATION

(11) **EP 4 205 671 B1**
(45) Date of publication and mention of the grant of the patent: **12.03.2025**
(21) Application number: 22217254.6
(22) Date of filing: 30.12.2022
(51) Int. Cl.: A61B 17/12

(54) **MEDICAL DEVICE DELIVERY SYSTEMS WITH TWISTING LOOP WIRES**
SYSTEME ZUR EINFÜHRUNG EINER MEDIZINISCHEN VORRICHTUNG MIT TWIST-LOOP-DRÄHTEN
SYSTÈMES DE POSE DE DISPOSITIF MÉDICAL AVEC FILS À BOUCLE DE TORSION

(30) Priority: 31.12.2021 US 202117566907
(43) Date of publication of application: 05.07.2023
(73) Proprietor: DePuy Synthes Products, Inc., Raynham, MA 02767 (US)
(72) Inventor: GOROCHOW, Lacey, Raynham, 02767 (US); SLAZAS, Robert, Raynham, 02767 (US); CHEN, Zhixian, Raynham, 02767 (US)
(74) Representative: Carpmaels & Ransford LLP

(56) References cited:
- EP-A1- 3 560 439
- WO-A1-2016/014985
- US-A1- 2008 119 887
- US-A1- 2016 278 782
- US-A1- 2021 338 248

## Description

### FIELD OF INVENTION

This disclosure generally relates to intravascular medical device systems that navigate through body vessels of a human subject and, more particularly, to detachment/delivery systems for delivering and deploying an implantable medical device to a target location of a body vessel.

### BACKGROUND

Aneurysms can be intravascularly treated by delivering a treatment device to the aneurysm to fill the sac of the aneurysm with embolic material and/or block the neck of the aneurysm to inhibit blood flow into the aneurysm. When filling the aneurysm sac, the embolic material can promote blood clotting to create a thrombotic mass within the aneurysm. When treating the aneurysm neck without substantially filling the aneurysm sac, blood flow into the neck of the aneurysm can be inhibited to induce venous stasis in the aneurysm and facilitate natural formation of a thrombotic mass within the aneurysm.

In some current treatments, multiple embolic coils are used to either fill the aneurysm sac or treat the entrance of the aneurysm neck. A common challenge among embolic coil treatments is that implanted coils and implanted portions of partially implanted coils can become entangled and difficult to reposition. In some instances, a physician may not be able to retract a partially implanted coil and may be forced to position the coil in a non-ideal location. Improperly positioning embolic coils at the aneurysm neck can potentially have the adverse effect of impeding the flow of blood in the adjoining blood vessel, particularly if the entrance and/or sac is overpacked. If a portion of the improperly placed coil becomes dislodged, it can enter the neighboring blood vessel and promote clot formation, which can ultimately lead to an obstruction that is tethered to the aneurysm and therefor extremely difficult to treat. Conversely, if the entrance and/or sac is insufficiently packed, blood flow can persist into the aneurysm.

In some current treatments, an embolic coil is attached to a tubular delivery device and delivered via a delivery catheter to an aneurysm. During delivery, the embolic coil can be engaged to the delivery member's implant detachment/deployment system (referred to herein equivalently as an "detachment system" or "deployment system"). When the embolic coil is in position, the deployment system can release the coil, the coil can be left implanted, and the delivery member can be retracted. Some treatments utilize a mechanical detachment/deployment system that can be actuated by a physician to release the implant by pulling one or more wires or other elongated members referred to generically herein as a "pull wire." Some of the challenges that have been associated with delivering and deploying embolic coils with delivery members having mechanical detachment systems include premature release of a coil due to premature movement of the pull wire proximally, thereby releasing the coil before the system is at the treatment site. This is exacerbated because of the system moves through tortuous vasculature to the treatment site.

There is therefore a need for improved methods, devices, and systems to facilitate implantation of embolic coils and other implants facing similar challenges.

The disclosure of EP3560439A1 provides a detachment system for delivering an implantable medical device to a target location of a body vessel that has a generally hollow distal tube. The distal tube includes a proximal end, a distal end, and a compressible portion of the tube itself, between the proximal and distal ends which is axially movable from a compressed to an elongated condition. A generally hollow proximal tube has a proximal end and a distal end. An engagement system engages and deploys the implantable medical device engaged at the distal end of the distal tube. The engagement system moves the compressible portion to the compressed condition when engaging the implantable medical device, and deploys the implantable medical device and releases the compressible portion to the elongated condition.

The disclosure of WO2016/014985A1 relates to embolic implants for use in the minimally-invasive treatment of aneurysms and other vascular disorders.

### SUMMARY

The present invention is defined in the appended claims. In some examples presented herein, premature proximal movement or translation of a pull wire can be decreased by providing a loop wire that twists one or more times around the pull wire, thereby providing a greater amount of friction against the pull wire.

A detachment system for delivering an implantable medical device to a target location of a body vessel can include a tubular body comprising a lumen extending therethrough and a compressed distal tube. The detachment system can include a loop wire comprising a first end attachment and a second end attachment affixed to the tubular body. The loop wire can further include a loop opening positioned proximate a distal end of the compressed distal tube. The detachment system can include a pull wire extending through the lumen and through the loop opening. The loop wire can include a twist such that that the loop wire is twisted at least one time around the pull wire to increase friction between the loop wire and the pull wire.

The loop wire can be twisted a single time around the pull wire, while in other examples the loop wire can be twisted a plurality of times around the pull wire. Additional twists can increase the friction/tightness of the junction between the pull wire and the loop wire.

The loop wire can include a friction coating proximate the pull wire to increase the friction between the pull wire and loop wire.

The loop wire and the pull wire can be movable to release the implantable medical device from the detachment system.

The twist can inhibit premature detachment of the implantable medical device by inhibiting proximal translation of the pull wire due to frictional resistance provided by the loop wire via the twist.

The tubular body can include a flexible coil disposed in a proximal direction from the compressed distal tube. The loop wire can inhibit elongation of the flexible coil when looped over the pull wire.

The detachment system can include a key affixed to the implantable medical device proximate a proximal end of the implantable medical device. The detachment system can include a stretch resistant fiber engaged to the key, extended through an implant lumen of the implantable medical device, and affixed to the implantable medical device proximate a distal end of the implantable medical device. The key can include a distal opening therethrough, wherein the stretch resistant fiber passes through the distal opening. The key can include a proximal opening therethrough. The key can include a bridge separating the distal opening and the proximal opening. The bridge can support a portion of the pull wire in a distal direction from the loop opening. The twist can be positioned on the loop wire proximal to the bridge.

The pull wire can be weaved across the bridge such that the pull wire passes from a first side of the key, through the proximal opening to a second side of the key and across the bridge, and through the distal opening to the first side of the key.

A detachment system for delivering an implantable medical device to a target location of a body vessel can include a pull wire extending through a tubular body of the detachment system. The detachment system can include a loop wire looped over the pull wire at a distal end of the loop wire and twisted at least once around the pull wire. A twist in the loop wire can inhibit premature detachment of the implantable medical device by inhibiting proximal translation of the pull wire relative to a loop opening in the distal end of the loop wire.

The loop wire can be twisted a single time around the pull wire, while in other examples the loop wire can be twisted a plurality of times around the pull wire. Additional twists can increase the friction/tightness of the junction between the pull wire and the loop wire.

The detachment system can include a compressed distal tube. The tubular body can include a flexible coil disposed in a proximal direction from the compressed distal tube. The loop wire can inhibit elongation of the flexible coil when looped over the pull wire.

The detachment system can include a key affixed to the implantable medical device proximate a proximal end of the implantable medical device. The detachment system can include a stretch resistant fiber engaged to the key, extended through an implant lumen of the implantable medical device, and affixed to the implantable medical device proximate a distal end of the implantable medical device. The key can include a distal opening therethrough, wherein the stretch resistant fiber can pass through the distal opening. The key can include a proximal opening therethrough. The key can include a bridge separating the distal opening and the proximal opening. The bridge can support a portion of the pull wire in a distal direction from the loop opening. The twist can be positioned on the loop wire proximal to the bridge.

A method for constructing a detachment system with an embolic implant and optionally deploying the implant, wherein the method for deploying the implant does not form part of the present invention, can include providing a tubular body comprising a lumen extending therethrough and a compressible distal tube. The method can include affixing a loop wire to the tubular body. The method can include compressing the compressible distal tube. The method can include positioning a loop opening in the loop wire proximate a distal end of the compressible distal tube such that the loop wire is extended through the lumen. The method can include extending a pull wire through the lumen. The method can include extending the loop opening through a key of an implantable medical device. The method can include twisting the loop wire around the pull wire at least one time. The method can include extending a distal end of the pull wire through the loop opening of the twisted loop wire.

The method to deploy the implant does not form part of the present invention and can include inhibiting, via frictional resistance of the twisted loop wire around the pull wire, translation of the pull wire through the loop wire while the implantable medical device is delivered through vasculature to a treatment site. The method can include overcoming frictional resistance such that the pull wire translates proximally and releases the implantable medical device at the treatment site.

### BRIEF DESCRIPTION OF THE DRAWINGS

The above and further aspects of this disclosure are further discussed with reference to the following description in conjunction with the accompanying drawings, in which like numerals indicate like structural elements and features in various figures. The drawings are not necessarily to scale, emphasis instead being placed upon illustrating principles of the disclosure. The figures depict one or more implementations of the inventive systems and devices, by way of example only, not by way of limitation.
FIG. 1A is an illustration of a delivery/detachment system and implant, according to the present invention.
FIG. 1B is a detailed view of a detachment system showing a twist in the loop wire proximate the detachment feature (i.e., key), according to the present invention.
FIG. 1C is another view of a detachment system wherein the loop wire is anchored/attached to the system at a distal tube, according to the present invention:
FIGs. 2A and 2B are illustrations of detachment features (i.e., keys) each having a stretch resistant fiber therethrough, according to aspects of the present disclosure;
FIGs. 3A-3C are illustrations of detachment features affixed to an embolic coil, according to aspects of the present disclosure;
FIG. 4 is an illustration of embolic coils being positioned within an aneurysm, according to aspects of the present disclosure;
FIGs. 5A-5B are illustrations of example loop wire variations that increase friction at a pull wire, according to the present invention
FIG. 5C is an illustration of an example loop wire variation that increases friction at a pull wire, according to aspects of the present disclosure;
FIGs. 6A-6D illustrate a sequence of steps for releasing an embolic implant from a detachment system, according to aspects of the present disclosure;
FIG. 7 is a flow diagram illustrating steps for designing, constructing, or configuring a detachment system and implant, according to aspects of the present disclosure;
FIG. 8A is an illustration of a design for a detachment feature (i.e., key) that enables the pull wire to weave around a bridge, according to aspects of the present disclosure; and
FIG. 8B is an illustration of a design for a detachment feature (i.e., key) that enables the pull wire to weave around multiple bridges, according to aspects of the present disclosure.

### DETAILED DESCRIPTION

An object of the present disclosure is to decrease the occurrence of or ultimately prevent premature detachment of an embolic coil from a detachment system prior to placing the coil at a treatment site, i.e., an aneurysm. More specifically, it is an object of the present disclosure to provide additional support to the junction between a loop wire and a pull wire of the detachment system. Certain current designs for embolic coil delivery systems can include a tubular body having a compressed distal tube that, once released from compression, delivers the embolic coil to a treatment site. Within that distal tube (also referred to herein as a "distal hypotube") runs both a loop wire and a pull wire. The loop wire can extend into a detachment features (also referred to herein as a "key") of the implant and loop onto the pull wire to secure the distal hypotube into its compressed state, while also containing the embolic coil that is attached to the key. One common pitfall to prior designs is that there is a chance that the pull wire can prematurely translate proximally from the loop wire, for example, as a physician is delivering the device through tortuosity and reactive frictional forces cause the pull wire to retract. Premature detachment of the detachment system from the embolic coil can be a significant problem, as the physician no longer controls the timing of the detachment of the embolic coil into the aneurysm. The present devices, systems, and methods provide a solution to early, inadvertent deployment of the embolic coil.

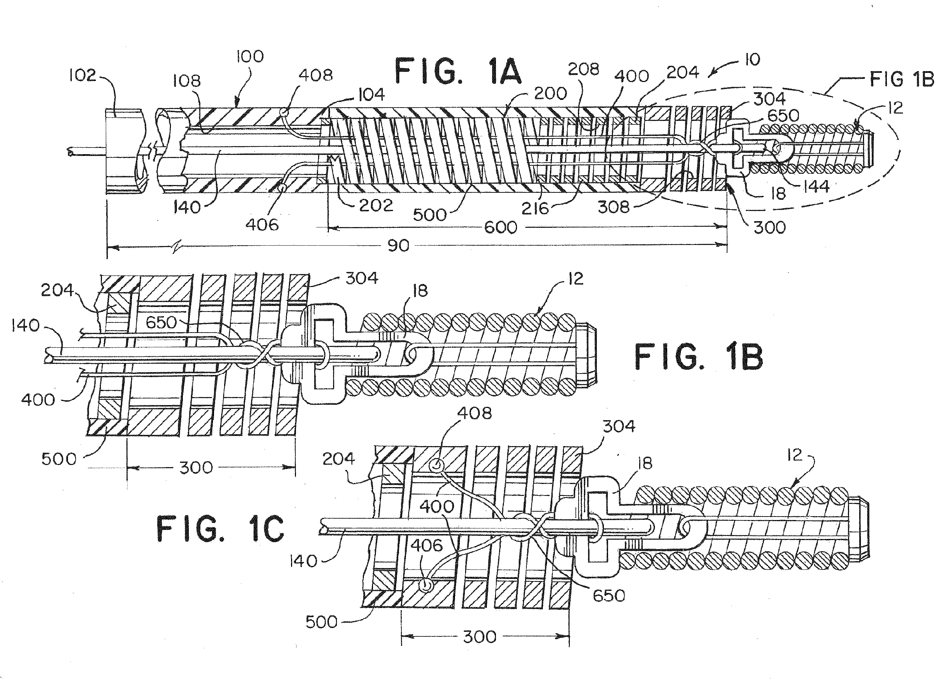

Referring to the figures, FIG. 1A is an illustration of a delivery/detachment system 10 and an implantable medical device 12 (which is an embolic coil in the example shown), according to the present invention. The implantable medical device 12 is also referred to herein as implant 12. The detachment system 10 can include a proximal tube 100, a coiled section 600 comprising a support coil 200, a distal tube 300, a sleeve 500 surrounding the coiled section 600, a loop wire 400 extending through the coiled section 600, and a pull wire 140 extending through the coiled section 600. A distal end 144 of the pull wire 140 can extend at least partially beyond a proximal portion of a key 18 (also referred to herein as "detachment feature") of the implant 12. The detachment system 10 can have a tubular body 90 that is formed by the proximal tube 100, the coiled section 600 comprising the support coil 200, and the distal tube 300. When the distal tube 300 is compressed, as will be described below for when the distal hypotube 300 includes a compressible portion 306, the distal tube 300 can be referred to as a compressed distal tube.

A proximal end 102 of the proximal tube 100 can extend proximally within a delivery member (e.g., catheter 250). A distal end 104 of the proximal tube 100 can be connected to a proximal end 202 of the support coil 200. A distal end 204 of the support coil 200 can be connected to the distal tube 300 at one end, and the implant 12 can be connected to the distal tube 300 at the distal end 304 of the distal tube 300. The proximal tube 100 can include a proximal lumen 108, the coiled section 600 and support coil 200 can include a coil lumen 208, and the distal tube 300 can include a distal lumen 308. The proximal lumen 108, coil lumen 208, and distal lumen 308 provide a contiguous lumen through which the pull wire 140 and loop wire 400 pass.

The coiled section 600 can be formed primarily of a non-radiopaque material, such as steel, and can include a radiopaque section 216 made of a radiopaque material, such as platinum and/or tungsten. The radiopaque section 216 can be positioned between a proximal, non-radiopaque section of the support coil 200 and a distal, non-radiopaque section of the support coil 200. The radiopaque section 216 can be positioned a predetermined distance from a distal end 304 of the detachment system 10 so that a physician can readily visualize the placement of the distal portion of the system during a treatment procedure. The proximal section, radiopaque section 216, and distal section of the support coil 200 can be concentrically welded.

The sleeve 500 can cover at least a portion of the flexible section 106 to inhibit deformation of the flexible section and/or reduce friction with vasculature and the flexible section 106 during intravascular navigation. In some examples, the sleeve 500 can cover about 10 cm of the proximal tube 100 approximate and/or including the distal end 104 of the proximal tube 100. When the detachment system 10 is assembled, the coiled section 600 and sleeve 500 can be more flexible than the distal hypotube 300 and the proximal hypotube 100. One way to measure flexibility is to perform a three-point bend test wherein a portion of the detachment system 10 is held fixed at two end points, a force is applied perpendicularly to the detachment system 10 centrally between the points, and flexibility is quantified by the length of deflection of the detachment system 10 caused by the force. When measured this way, in some examples, the coiled section 600 and sleeve 500 can be about 1.5 times more flexible than the distal hypotube 300 and about 20 times more flexible than the proximal hypotube 100. In other words, when the three-point test is performed identically on the three sections 100, 600, 300, the coiled section 600 can deflect over a length that is about 1.5 time the deflection length of the distal hypotube 300 and about 20 times the length of deflection of the proximal hypotube 100. Flexibility can be measured in other ways as would be appreciated and understood by a person of ordinary skill in the art. When the detachment system 10 is assembled, the coiled section 600 and sleeve 500 can be more flexible than the distal hypotube and the proximal hypotube as flexibility is determined by other means as would be known to a person of ordinary skill in the art.

The loop wire 400 can be attached to the detachment system 10 at locations along the tubular body 90. The loop wire 400 can include a first end attachment 406 to connect the loop wire 400 to the wall of the lumen 108, 208, 308 and a second end attachment 408 to connect an opposite end of the loop wire 400 to the wall of the lumen 108, 208, 308. The first end attachment 406 and second end attachment 408 can be welds, adhesives, or other mechanical fasteners that connect the loop wire 400 to the tubular body 90. The first end attachment 406 and second end attachment 408 can be located along the proximal hypotube 100, as shown in FIG. 1A, or any other location of the tubular body 90, including along the coiled section 600 or the proximal hypotube 300.

The loop wire 400 can include a twist 650 to provide additional resistance/friction between the loop wire 400 the pull wire 140, as described above and shown in FIG. 1A. FIG. 1B provides a detailed view of a detachment system 10 showing the twist 650 in the loop wire 400 proximate a detachment feature (i.e., key 18), according to the present invention. In prior system, the loop wire 400 is at one side of the pull wire 140 proximal to the key 18. After passing a proximal end of the key 18, the loop wire 400 loops around the key 18 and around the pull wire 140 to the other side of the loop wire 140, thereby securing the key 18 to the pull wire 140. The key 18 (and implant 12) can be deployed from the detachment system 10 by translating the pull wire 140 proximally, beyond the loop in the loop wire 400. In FIG. 1B, the first end attachment 406 and second end attachment 408 are located proximal on the device along tubular body 90. FIG. 1C provides an example of a loop wire 400 that is anchored/attached to the tubular body 90 along the distal tube 300. For example, the first end attachment 406 and second end attachment 408 are positioned along the distal tube 300, which is in accordance with some embodiments.

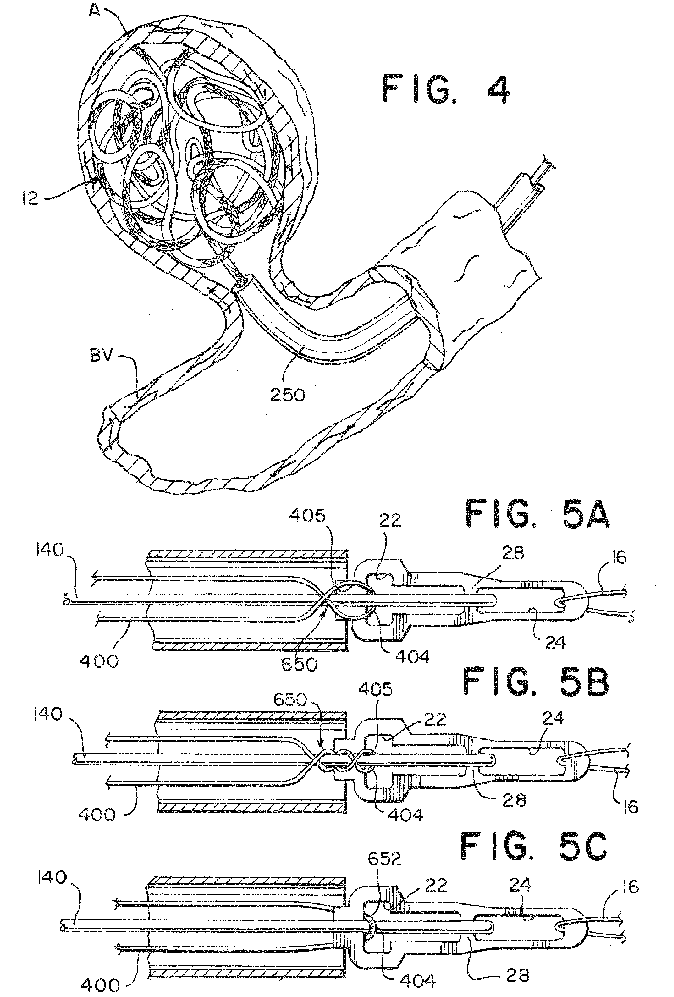

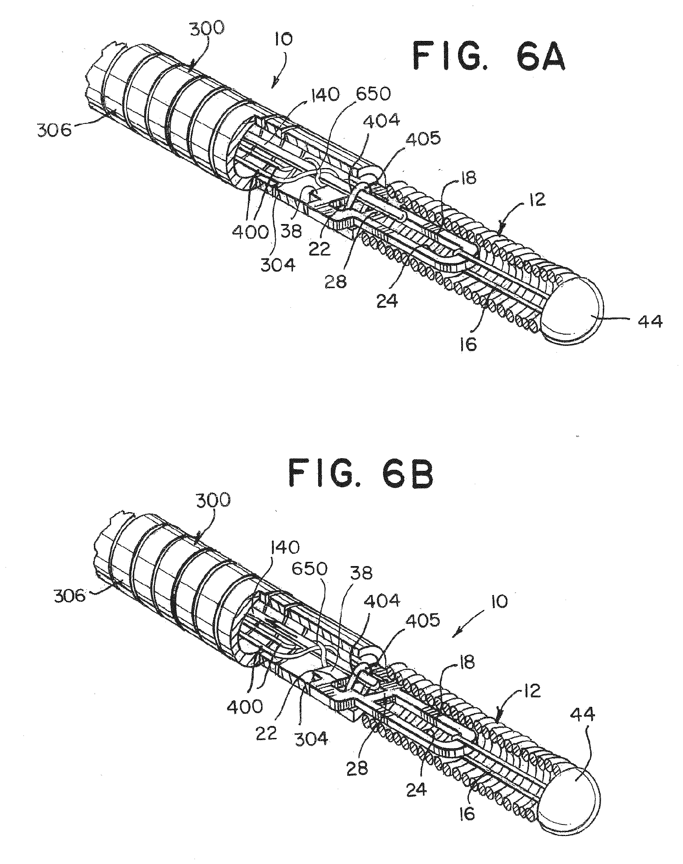

As described above, one common concern with prior systems is inadvertent proximal translation of the pull wire as the system is deliver through the tortuosity. By twisting the loop wire 400 around the pull wire 140 one or more times, additional resistance prevents inadvertent proximal translation of the pull wire. Additional details and variations of the twist 650 are provided in the discussion of FIGs. 5A-5C. The distal tube 300 can be compressed or a portion of the distal tube 300 can be compressed such that, once the pull wire 140 is removed from the loop at the end of the loop wire 400, the compressed portion of the distal tube 300 can expand to deliver the implant 12. FIGs. 6A-6B provide a detailed view of a compressible portion 306 of the distal hypotube 300.

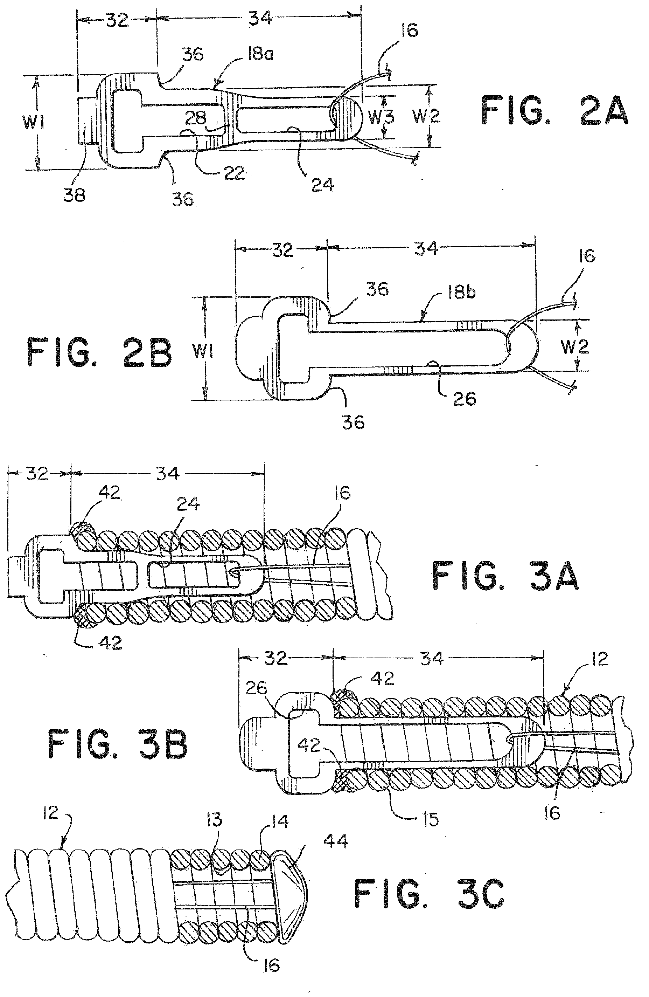

FIGs. 2A and 2B are illustrations of detachment features (i.e., keys 18) each having a stretch resistant fiber 16 therethrough, according to aspects of the present disclosure. FIG. 2A illustrates a dual opening key 18a having a proximal portion 32 that is sized to engage a mechanical detachment system 10 and/or delivery tube (e.g., the distal hypotube 300). The proximal portion 32 is illustrated as having a width W1. The dual opening key 18a can have a distal portion 34 that is sized to fit within a lumen 13 of the embolic coil (e.g., implant 12). The distal portion 34 can have a wider section having a width W2 that is about as wide as the inner diameter of the implant 12 and a tapered section having a width W3 that is narrower than the inner diameter of the implant 12. The dual opening key 18a can have a proximal tab 38 that is narrower than the proximal portion 32 and is sized to fit within a lumen of a delivery tube (e.g., distal lumen 308). The "dual opening" of the dual opening key 18a can refer to the two separate openings within the face of the key 18a, for example a proximal opening 22 and a distal opening 24. A bridge 28 can separate the proximal opening 22 and the distal opening 24, as illustrated. The bridge 28 can be used to support the distal end 144 of the pull wire 140 when the detachment system 10 is in the loaded/pre-deployed state.

FIG. 2B illustrates a single opening key 18b having a proximal portion 32 that is sized to engage a mechanical detachment system 10 and/or delivery tube (e.g., the distal hypotube 300). The proximal portion 32 is illustrated having a width W1. The single opening key 18b can have a distal portion 34 narrower than the proximal portion 32 and sized to fit within the lumen 13 of the implant 12. The single opening key 18b can have a proximal tab 38 that is narrower than the proximal portion 32 and sized to fit within a lumen of a delivery tube, as also shown for the dual opening key 18a.

When reference is made herein to a key 18, it will be understood to include a dual opening key 18a or a single opening key 18b. After the key 18 is formed, a stretch resistant fiber 16 can be threaded through a distal opening 24 of the dual opening key 18a or the single opening 26 of the single opening key 18b. The stretch resistant fiber, which can be a suture material and the like, can secure the key to the embolic coil portion of the implant. The key 18 can include engagement surfaces 36 at a distal end of the proximal portion 32 of the key 18. This engagement surfaces 36 can abut a proximal end 15 of the implant 12.

FIGs. 3A-3C are illustrations of keys 18 affixed to an embolic coil (e.g., implant 12), according to aspects of the present disclosure. In particular, FIGs. 3A and 3B are illustrations of the keys 18 with the distal portion 34 fully inserted into the lumen 13 of the implant 12 and wherein the key 18 is affixed to the implant 12 with welds 42 or other attachments. The welds 52 can be positioned at locations wherein the engagement surfaces 36 of the key 18 meets the proximal end 15 of the implant 12. In both FIGs. 3A and 3B, the key 18 is illustrated having a distal portion 34 that has a width over at least a portion of the length of the distal portion 34 that is about equal to the inner diameter of the lumen 13 of the implant 12.

FIG. 4 is an illustration of embolic coils (e.g., implant 12) being positioned within an aneurysm A, according to aspects of the present disclosure. The detachment system 10 is passed through a blood vessels BV to the aneurysm A through a catheter 250. Once positioned, the implant(s) 12 can loop and bend within the aneurysm sac to form a thrombotic mass. The implant(s) 12 can loop back on themselves and/or loop next to other implants. As the aneurysm A becomes increasingly packed, overlapping portions of the implant 12 can press into each other.

FIGs. 5A-5C are illustrations of example loop wire 400 variations that increase friction at a pull wire 140. FIGs**.** 5A and 5B specifically show variations of twist 650 of the loop wire 400 at a distal end 144 of the pull wire 140. As stated above, the loop wire 400 can extend through the lumen (e.g., lumen 108, 208, 308) of the tubular member on one side of the pull wire 140. After the loop wire passes the proximal end of the key 18 (e.g., the proximal extension 38), the loop wire 400 can cross over the pull wire 140 such and a distal end 404 of the loop wire 400 forms an opening 405 through which the pull wire 140 passes.

The present detachment system 10 can include a twist 650 in the loop wire proximal to where the loop wire 400 crosses over the pull wire 400. The twist 650 causes the loop wire 400 to pass around the pull wire at least one time before terminating at the final, distal opening 405 of the pull wire 400. This twist 650 can be a singular twist, as shown in FIG. 5A, or the twist 650 can be a plurality of twists, as shown in FIG. 5B. The one or more twists 650 can increase the degree of tightness at the distal end 144 of the pull wire 140, thereby preventing unwanted proximal translation and resultant premature deployment of the implant 12. As described above, when the key is a dual opening key 18a, the distal end 144 of the pull wire 140 can be supported by the bridge 28 between the proximal opening 22 and the distal opening 24. The twist 650, in these examples, can be positioned proximal to the bridge 28, and the opening 405 in the loop wire 400 can be positioned within the proximal opening 22. When the key is a single opening key 18b, the opening 405 of the loop wire 400 can be within the singular opening 26.

Referring to FIG. 5C, example detachment systems 10 can include a friction coating 652 on the distal end 404 of the loop wire 400 proximate the opening 405 in the loop wire 400 through which the pull wire 140 extends. The friction coating 652 can include a silicone, rubber, synthetic polymer, or other coating to increase friction at the junction between the pull wire 140 and the loop wire 400. Alternatively, the distal end 404 of the loop wire 400 can have a roughened surface, for example by providing hatch marks and the like so as to increase the friction at the junction between the pull wire 140 and the loop wire 400. In some examples, the distal end 144 of the pull wire 140 can also be configured to create additional friction. In certain prior system designs, a low-friction polytetrafluoroethylene (PTFE) coating may be applied to the pull wire 140 to decrease friction to enable easier deployment of the implant 12. However, easy deployment can, in some cases, cause inadvertent deployment of the implant. It is contemplated that the distal end 144 of the pull wire 140 does not include a PTFE coating. In some examples, the distal end 144 of the pull wire 140 can include a silicone, rubber, synthetic polymer, or other coating to increase friction at the junction between the pull wire 140 and the loop wire 400. Alternatively, the distal end 144 of the pull wire 140 can have a roughened surface, for example by providing hatch marks and the like so as to increase the friction at the junction between the pull wire 140 and the loop wire 400.

FIGs. 6A-6D illustrate a sequence of steps for releasing an embolic implant 12 from a detachment system 10, according to aspects of the present disclosure. FIG. 6A is an illustration of the implant 12 and delivery tube (e.g., distal hypotube 300) configured for delivery and positioning of the implant 12. FIGs. 6B through 6D illustrate releasing the example embolic implant 12 from the distal hypotube 300. A portion of the distal hypotube 300 is cut away for illustration purposes. The more proximal features of the tubular body 90 are not shown in the views.

FIG. 6A illustrates the detachment system including a pull wire 140 and a loop wire 400 locked into the key 18 of the implant 12 (the key shown in FIGs. 6A-6D is a dual opening key 18a, but the illustrations could equally apply to a single opening key 18b or a triple opening key 18c). The distal tube 300 can include a compressible portion 306. The loop wire 400 can have an opening 405 at a distal end 404 of the loop wire 400, and the opening 405 can be placed through an opening in the key 18 (e.g., proximal opening 22 in a dual opening key 18a, or the singular opening 26 in a single opening key 18b). As described above, the twist 650 can be twisted around the pull wire 140 proximal to the terminating end, i.e., the opening 405 through which the pull wire 140 extends. When the pull wire 140 is placed through the opening 405, the implant 12 is now secure.

In the case of a dual opening key 18a, the key can include a bridge 28 positioned distally from the loop wire opening 405 and positioned to support a distal portion of the pull wire 140 that is distal of where the loop wire opening 405 wraps around by the pull wire 140. Configured thusly, the bridge 28 can support the distal portion of the pull wire 140 such that when the loop wire 400 tensions against the pull wire 140 at the loop opening 405, the bridge 28 can inhibit the distal portion of the pull wire 140 from deforming. The proximal tab 38 of the key 18 can be positioned to support a portion of the pull wire 140 that is proximal of where the loop wire opening 405 is supported by the pull wire 140. The combination of the bridge 28 and the proximal tab 38 can inhibit the pull wire 140 from deforming due to forces applied by the loop wire 400. The distal hypotube 300 can be detachably attached to the implant 12 as illustrated in FIG. 6A during delivery of the implant 12 through the vasculature and while the implant 12 is being positioned at a treatment site. The bridge 28 can reduce the likelihood that the implant 12 is prematurely released due to bending of the pull wire 140 due to forces from the loop wire 400.

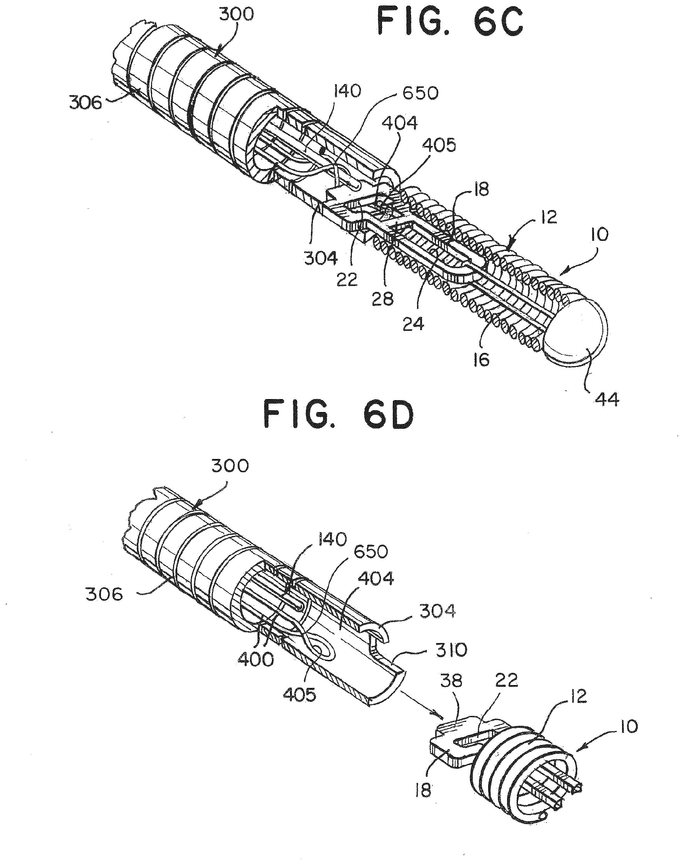

FIG. 6B illustrates the pull wire 140 being drawn proximally to begin the release sequence for the implant 12. FIG. 6C illustrates the instant the pull wire 140 exits the opening 405 and is pulled free of the loop wire 400. The distal end 404 of the loop wire 400 falls away and exits the key 18. As can be seen, there is now nothing holding the implant 12 to the distal hypotube 300. FIG. 6D illustrates the end of the release sequence. Here, the compressible portion 306 has expanded/returned to its original shape and "sprung" forward. An elastic force E is imparted by the distal end 304 of the distal hypotube 300 to the implant 12 to "push" it away to ensure a clean separation and delivery of the implant 12. The compressible portion 306 can be a spiral cut portion of the distal hypotube 300, for example a laser cut spiraled segment that can be compressed when the detachment system 10 is loaded.

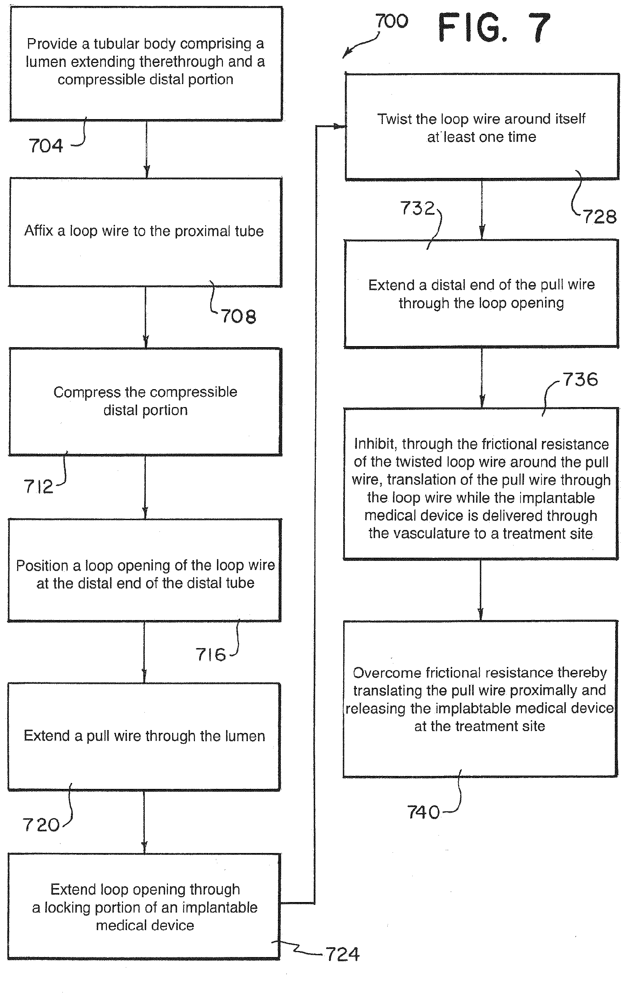

FIG. 7 is a flow diagram illustrating a method 800 for designing, constructing, or configuring a detachment system 10 and implant 12, according to aspects of the present disclosure. Steps 704 through 732 describe steps to create/construct one or more of detachment systems 10 described herein. In step 704, the construction of the detachment system 10 can begin with providing a tubular body 90 comprising a lumen (e.g., lumen 108, 208, 308) extending therethrough and a compressible distal tube (e.g., distal hypotube 300). In step 708, a loop wire 400 can be affixed to the tubular body 90. For example, proximal ends of the loop wire can be attached to the tubular body at a first end attachment 406 and a second end attachment 408.

In step 712, the compressible distal tube 300 can be compressed into its loaded configuration. At step 716, a loop wire opening 405 in the loop wire 400 can be positioned proximate a distal end 304 of the compressible distal tube such that the loop wire 400 is extended through the lumen (e.g., lumen 108, 208, 308). In step 720, the pull wire 140 can be extended through the lumen (e.g., lumen 108, 208, 308).

In step 724, the loop opening 405 can be extended through a key 18 of an implantable medical device 12. In step 728, the loop wire 400 can be twisted around the pull wire 140 at least one time, thereby creating the loop wire twist 650 described herein. In step 732, a distal end 144 of the pull wire 140 can be extended through the twist 650 and loop opening 405 of the twisted loop wire 140.

The steps for creating/constructing the detachment system 10 can end after step 732. In some examples, steps 736 and 740 provide additional steps to inhibit inadvertent proximal translation of the pull wire 140 and such that the implant can be deployed. For example, in step 736, not forming part of the present invention, proximal translation of the pull wire 140 through the loop wire 400 while the implantable medical device 12 is delivered through vasculature to a treatment site can be inhibited via frictional resistance of the twisted loop wire 400 around the pull wire 140. In step 740, not forming part of the present invention, frictional resistance of the junction between the loop wire 400 and pull wire 140 can be overcome such that the pull wire 140 translates proximally and releases the implantable medical device 12 at the treatment site.

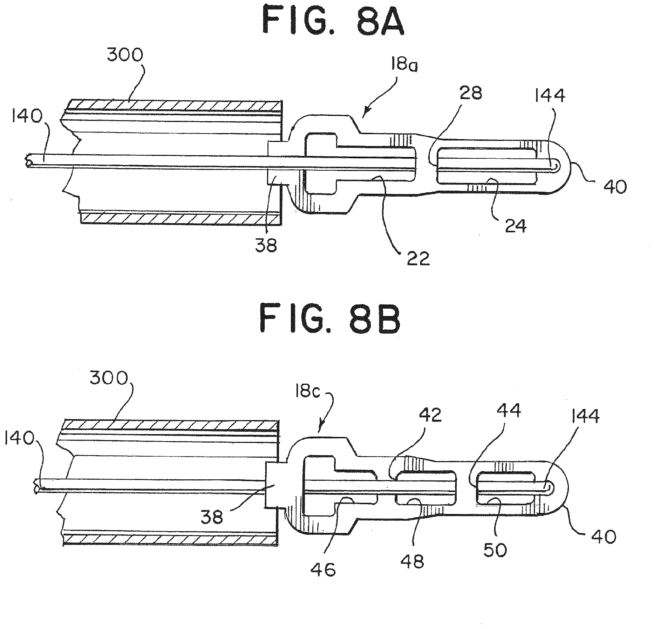

In some examples, the key 18 can be designed to provide additional friction at the pull wire 140 so as to further inhibit premature proximal translation of the pull wire 140. FIGs. 8A and 8B provide examples of such a construct, wherein the pull wire 140 can be weaved around one or more bridges of the key 18. FIG. 8A shows a "dual opening" key 18a, as shown and described above with reference to FIG. 2A. The key 18a can include a bridge 28 to separate the proximal opening 22 and the distal opening 24, as illustrated. In some examples, the pull wire 140 can be weaved around the bridge 28 to create additional friction at the junction between the pull wire 140 and key 18a. For example and as shown in FIG. 8A, the pull wire 140 can pass over the proximal tab 38 of the key 18a at a first side of the key 18a, pass over/under the bridge 28 on a second side of the key 18a, weave back to the first side of the key 18a, and rest upon a distal end 40 of the key 18a on the first side of the key 18a. In weaving the pull wire 140 around the bridge 28, the pull wire 140 can have a stronger engagement with the key 18a so as to inhibit inadvertent proximal translation of the pull wire 140.

In some examples, the key 18 can include a plurality of bridges to enable more weaving of the pull wire 140 and thus provide additional friction. Referring to FIG. 8B, the key 18c (e.g., a triple-opening key) includes a first bridge 42 and a second bridge 44. The first bridge 42 creates a first opening 46 between the proximal tab 38 and the first bridge 42. The second bridge 44 creates a second opening 48 between the first bridge 42 and the second bridge 44, as well as a third opening 50 between the second bridge 44 and the distal end 40 of the key 18c. In this example design, the pull wire 140 can weave around the first bridge 42 and second bridge 44 through the first opening 46, the second opening 48, and the third opening 50. To illustrate, the pull wire 140 can pass over the proximal tab 38 of the key 18c at a first side of the key 18a, pass over/under the first bridge 42 on a second side of the key 18c, pass under/over the second bridge 44 on the first side of the key 18c, weave back to the second side of the key 18c, and rest upon a distal end 40 of the key 18c on the second side of the key 18c. It will be understood that the weaved pull wire 140 described with reference to FIGs. 8A and 8B can be used along with the twisting loop wires 400 described herein; it will also be understood that the weaved pull wire 140 described with reference to FIGs. 8A and 8B can be implemented as an alternative to the twisting loop wires 400 as a means to increase friction.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

As described herein, the disclosure contemplates many variations and modifications of the implant and methods for making and using the same, wherein the methods for using the same do not form part of the present invention, including alternative materials, alternative geometries of component parts, alternative positioning of component parts in relation to each other, etc. These modifications would be apparent to those having ordinary skill in the art to which this disclosure relates. The scope of the invention is defined by the claims which follow.

## Claims

1. A detachment system (10) for delivering an implantable medical device (12) to a target location of a body vessel, the system comprising:
a tubular body (90) comprising a lumen (108, 208, 308) extending therethrough and a compressed distal tube (300);
a loop wire (400) comprising a first end attachment (406) and a second end attachment (408) affixed to the tubular body and further comprising a loop opening (405) positioned proximate a distal end (304) of the compressed distal tube; and
a pull wire (140) extending through the lumen and through the loop opening,
**characterised in that** the loop wire comprises a twist (650) such that that the loop wire is twisted at least one time around the pull wire to increase friction between the loop wire and the pull wire.

2. The detachment system of claim 1, wherein a distal end of the loop wire comprises a friction coating (652) proximate the pull wire.

3. The detachment system of claim 1, wherein the loop wire and the pull wire are movable to release the implantable medical device from the detachment system.

4. The detachment system of claim 1, wherein the twist is configured to inhibit premature detachment of the implantable medical device by inhibiting proximal translation of the pull wire due to frictional resistance provided by the loop wire via the twist.

5. The detachment system of claim 1, wherein the tubular body further comprises a flexible coil disposed in a proximal direction from the compressed distal tube, wherein the loop wire inhibits elongation of the flexible coil when looped over the pull wire.

6. The detachment system of claim 1, wherein the loop wire is twisted a plurality of times around the pull wire.

7. The detachment system of claim 6, wherein the detachment system comprises a compressed distal tube, wherein the tubular body comprises a flexible coil (200) disposed in a proximal direction from the compressed distal tube, wherein the loop wire inhibits elongation of the flexible coil when looped over the pull wire.

8. The detachment system of claim 1, further comprising:
a key (18) configured to be affixed to the implantable medical device proximate a proximal end (15) of the implantable medical device; and
a stretch resistant fiber (16) engaged to the key, configured to be extended through an implant lumen of the implantable medical device, and configured to be affixed to the implantable medical device proximate a distal end of the implantable medical device.

9. The detachment system of claim 8, wherein the key comprises:
a distal opening (24) therethrough, wherein the stretch resistant fiber passes through the distal opening;
a proximal opening (22) therethrough; and
a bridge (28) separating the distal opening and the proximal opening.

10. The detachment system of claim 9, wherein the bridge supports a portion of the pull wire in a distal direction from the loop opening.

11. The detachment system of claim 10, wherein the twist is positioned in the loop wire proximal to the bridge.

12. The detachment system of claim 9 when dependent on claim 1, wherein the pull wire is weaved across the bridge such that the pull wire passes from a first side of the key, through the proximal opening to a second side of the key and across the bridge, and through the distal opening to the first side of the key.

13. A method comprising:
providing a tubular body (90) comprising a lumen (108, 208, 308) extending therethrough and a compressible distal tube (300);
affixing a loop wire (400) to the tubular body;
compressing the compressible distal tube;
positioning a loop opening (405) in the loop wire proximate a distal end (304) of the compressible distal tube such that the loop wire is extended through the lumen;
extending a pull wire (140) through the lumen;
extending the loop opening through a key (18) of an implantable medical device (12);
twisting the loop wire around the pull wire at least one time; and
extending a distal end (144) of the pull wire through the loop opening of the twisted loop wire.

## Patentansprüche

1. Ablösesystem (10) zum Einbringen einer implantierbaren medizinischen Vorrichtung (12) an eine Zielstelle eines Körpergefäßes, das System umfassend:
einen röhrenförmigen Körper (90), umfassend ein Lumen (108, 208, 308), das sich dahindurch erstreckt, und eine komprimierte distale Röhre (300);
einen Schleifendraht (400), umfassend eine erste Endbefestigung (406) und eine zweite Endbefestigung (408), die an dem röhrenförmigen Körper angebracht sind, und ferner umfassend eine Schleifenöffnung (405), die in der Nähe eines distalen Endes (304) der komprimierten distalen Röhre positioniert ist; und
einen Zugdraht (140), der sich durch das Lumen und durch die Schleifenöffnung erstreckt, **dadurch gekennzeichnet, dass** der Schleifendraht eine Verdrehung (650) umfasst, derart, dass der Schleifendraht mindestens einmal um den Zugdraht gedreht ist, um eine Reibung zwischen dem Schleifendraht und dem Zugdraht zu erhöhen.

2. Ablösesystem nach Anspruch 1, wobei ein distales Ende des Schleifendrahtes in der Nähe des Zugdrahtes eine Reibungsbeschichtung (652) umfasst.

3. Ablösesystem nach Anspruch 1, wobei der Schleifendraht und der Zugdraht beweglich sind, um die implantierbare medizinische Vorrichtung von dem Ablösesystem freizugeben.

4. Ablösesystem nach Anspruch 1, wobei die Drehung konfiguriert ist, um eine vorzeitige Ablösung der implantierbaren medizinischen Vorrichtung zu verhindern, durch Verhindern einer proximalen Translation des Zugdrahts aufgrund eines Reibungswiderstands, der durch den Schleifendraht über die Verdrehung bereitgestellt wird.

5. Ablösesystem nach Anspruch 1, wobei der röhrenförmige Körper ferner eine flexible Spule umfasst, die in einer proximalen Richtung von der komprimierten distalen Röhre angeordnet ist, wobei der Schleifendraht eine Verlängerung der flexiblen Spule verhindert, wenn er über den Zugdraht geschlungen wird.

6. Ablösesystem nach Anspruch 1, wobei der Schleifendraht eine Vielzahl von Malen um den Zugdraht verdreht ist.

7. Ablösesystem nach Anspruch 6, wobei das Ablösesystem eine komprimierte distale Röhre umfasst, wobei der röhrenförmige Körper eine flexible Spule (200) umfasst, die in einer proximalen Richtung von der komprimierten distalen Röhre angeordnet ist, wobei der Schleifendraht die Verlängerung der flexiblen Spule verhindert, wenn er über den Zugdraht geschlungen wird.

8. Ablösesystem nach Anspruch 1, ferner umfassend:
einen Schlüssel (18), der konfiguriert ist, um an der implantierbaren medizinischen Vorrichtung in der Nähe eines proximalen Endes (15) der implantierbaren medizinischen Vorrichtung angebracht zu werden; und
eine dehnungsfeste Faser (16), die mit dem Schlüssel in Eingriff steht, die konfiguriert ist, um sich durch ein Implantatlumen der implantierbaren medizinischen Vorrichtung zu erstrecken, und konfiguriert ist, um in der Nähe eines distalen Endes der implantierbaren medizinischen Vorrichtung an der implantierbaren medizinischen Vorrichtung angebracht zu werden.

9. Ablösesystem nach Anspruch 8, wobei der Schlüssel umfasst:
eine distale Öffnung (24) hindurch, wobei die dehnungsbeständige Faser durch die distale Öffnung verläuft;
eine proximale Öffnung (22) hindurch; und
eine Brücke (28), die die distale Öffnung und die proximale Öffnung trennt.

10. Ablösesystem nach Anspruch 9, wobei die Brücke einen Abschnitt des Zugdrahtes in einer distalen Richtung von der Schleifenöffnung stützt.

11. Ablösesystem nach Anspruch 10, wobei die Drehung in dem Schleifendraht proximal zu der Brücke positioniert ist.

12. Ablösesystem nach Anspruch 9, sofern abhängig von Anspruch 1, wobei der Zugdraht derart über die Brücke gewebt ist, dass der Zugdraht von einer ersten Seite des Schlüssels durch die proximale Öffnung zu einer zweiten Seite des Schlüssels und über die Brücke und durch die distale Öffnung zu der ersten Seite des Schlüssels verläuft.

13. Verfahren, umfassend:
Bereitstellen eines röhrenförmigen Körpers (90), umfassend ein Lumen (108, 208, 308), das sich dahindurch erstreckt, und eine komprimierte distale Röhre (300);
Anbringen eines Schleifendrahts (400) an dem röhrenförmigen Körper;
Komprimieren der komprimierbaren distalen Röhre;
Positionieren einer Schleifenöffnung (405) in dem Schleifendraht in der Nähe eines distalen Endes (304) der komprimierbaren distalen Röhre, derart, dass sich der Schleifendraht durch das Lumen erstreckt;
Strecken eines Zugdrahtes (140) durch das Lumen;
Strecken der Schleifenöffnung durch einen Schlüssel (18) einer implantierbaren medizinischen Vorrichtung (12);
mindestens einmaliges Verdrehen des Schleifendrahtes um den Zugdraht; und
Strecken eines distalen Endes (144) des Zugdrahtes durch die Schleifenöffnung des verdrehten Schleifendrahtes.

## Revendications

1. Système de détachement (10) pour la pose d'un dispositif médical implantable (12) à un emplacement cible d'un vaisseau corporel, le système comprenant :
un corps tubulaire (90) comprenant une lumière (108, 208, 308) s'étendant à travers celui-ci et un tube distal comprimé (300) ;
un fil de boucle (400) comprenant un élément de fixation de première extrémité (406) et un élément de fixation de seconde extrémité (408) fixés au corps tubulaire et comprenant en outre une ouverture de boucle (405) positionnée à proximité d'une extrémité distale (304) du tube distal comprimé ; et
un fil de traction (140) s'étendant à travers la lumière et à travers l'ouverture de boucle, **caractérisé en ce que** le fil de boucle comprend une torsion (650) telle que le fil de boucle est torsadé au moins une fois autour du fil de traction afin d'augmenter une friction entre le fil de boucle et le fil de traction.

2. Système de détachement selon la revendication 1, dans lequel une extrémité distale du fil de boucle comprend un revêtement par friction (652) à proximité du fil de traction.

3. Système de détachement selon la revendication 1, dans lequel le fil de boucle et le fil de traction sont mobiles pour libérer le dispositif médical implantable du système de détachement.

4. Système de détachement selon la revendication 1, dans lequel la torsion est conçue pour empêcher un détachement prématuré du dispositif médical implantable en empêchant une translation proximale du fil de traction en raison d'une résistance à la friction fournie par le fil de boucle par le biais de la torsion.

5. Système de détachement selon la revendication 1, dans lequel le corps tubulaire comprend en outre une bobine flexible disposée dans une direction proximale à partir du tube distal comprimé, dans lequel le fil de boucle empêche un allongement de la bobine flexible lorsqu'elle est bouclée sur le fil de traction.

6. Système de détachement selon la revendication 1, dans lequel le fil de boucle est torsadé une pluralité de fois autour du fil de traction.

7. Système de détachement selon la revendication 6, dans lequel le système de détachement comprend un tube distal comprimé, dans lequel le corps tubulaire comprend une bobine flexible (200) disposée dans une direction proximale à partir du tube distal comprimé, dans lequel le fil de boucle empêche un allongement de la bobine flexible lorsqu'elle est bouclée sur le fil de traction.

8. Système de détachement selon la revendication 1, comprenant en outre :
une clé (18) conçue pour être fixée au dispositif médical implantable à proximité d'une extrémité proximale (15) du dispositif médical implantable ; et
une fibre résistante à l'étirement (16) en prise dans la clé, conçue pour être étendue à travers une lumière d'implant du dispositif médical implantable, et conçue pour être fixée au dispositif médical implantable à proximité d'une extrémité distale du dispositif médical implantable.

9. Système de détachement selon la revendication 8, dans lequel la clé comprend :
une ouverture distale (24) à travers celle-ci, dans lequel la fibre résistante à l'étirement passe à travers l'ouverture distale ;
une ouverture proximale (22) à travers celle-ci ; et
un pont (28) séparant l'ouverture distale et l'ouverture proximale.

10. Système de détachement selon la revendication 9, dans lequel le pont supporte une partie du fil de traction dans une direction distale de l'ouverture de boucle.

11. Système de détachement selon la revendication 10, dans lequel la torsion est positionnée dans le fil de boucle à proximité du pont.

12. Système de détachement selon la revendication 9 lorsque dépendante de la revendication 1, dans lequel le fil de traction est tissé à travers le pont de sorte que le fil de traction passe d'un premier côté de la clé, à travers l'ouverture proximale vers un second côté de la clé et à travers le pont, et à travers l'ouverture distale vers le premier côté de la clé.

13. Procédé comprenant :
la fourniture d'un corps tubulaire (90) comprenant une lumière (108, 208, 308) s'étendant à travers celui-ci et un tube distal compressible (300) ;
la fixation d'un fil de boucle (400) au corps tubulaire ;
la compression du tube distal compressible ;
le positionnement d'une ouverture de boucle (405) dans le fil de boucle à proximité d'une extrémité distale (304) du tube distal compressible de sorte que le fil de boucle soit étendu à travers la lumière ;
l'extension d'un fil de traction (140) à travers la lumière ;
l'extension de l'ouverture de boucle à travers une clé (18) d'un dispositif médical implantable (12) ;
la torsion du fil de boucle autour du fil de traction au moins une fois ; et
l'extension d'une extrémité distale (144) du fil de traction à travers l'ouverture de boucle du fil de boucle torsadé.
